# EUROPEAN PATENT APPLICATION

(11) **EP 1 921 451 A2**
(43) Date of publication of application: **14.05.2008**
(21) Application number: 08003771.6
(22) Date of filing: 22.05.2002
(51) Int. Cl.: G01N 33/555, G01N 33/569, A61K 35/18

(54) **Complement mediated assays for in vivo and in vitro methods**

(30) Priority: 22.05.2001 US 292616 P; 11.06.2001 US 297321 P; 21.09.2001 US 324029 P
(62) Divisional of application: 02806678.5
(71) Applicant: Cygene, Inc., Sunrise, FL 33351 (US)
(72) Inventor: Ramberg, Elliot R., Hollywood, FL 33024 (US)
(74) Representative: Polypatent

(57) **Abstract**

The present invention is directed to methods and compositions for detection of target analytes, comprising proteins and nucleic acids, in multiple cellular compartments. Preferred embodiments comprise the use of complement-mediated assays and complement mediated treatments for human and animals. Methods and compositions for nionitoring multiple stages of disease and infection are presented.

## Description

### FIELD OF THE INVENTION

The present invention is directed to methods and compositions for detecting pathological conditions. In particular, the invention comprises methods and compositions using biological factors, such as complement components, for detecting pathological conditions.

### BACKGROUND OF THE INVENTION

Diagnostics has traversed a broad range of disciplines from an initial foothold in serologic diagnostics to DNA molecular diagnostics, such as those using PCR. Problems with many current diagnostic technologies include the inability to directly detect species specific mRNA and proteins, and many also lack specificity and sensitivity. The problems of detection of molecular cancer metastasis, detection of residual disease, the early detection of HIV and other viral agents, sensitive carcinogen detection, sensitivity in detection of pathologic proteins or cells in normal tissue, and the need for heightened specificity and sensitivity in the determination of the precancerous state of dysplasia, illustrate the need for more accurate, sensitive and specific assays. Furthermore, most of these assays fail in detection of very low numbers of antigen or analyte targets, such as low number DNA, mRNA, protein or cellular targets in the presence of a large amount of non-specific material such as genomic DNA, mRNA, protein, or cells.

Other attempts at measuring infectious agents include the tests for prions. Creutzfeld-Jakob disease (CJD) of humans and bovine spongiform encephalitis (BSE) and scrapie of animals are neurodegenerative diseases caused by prion proteins. The infectious prion is an abnormal disease-producing isoform of the normal prion protein (PrP) called PrP^{SC}. Brain damage in prion disease occurs when abnormal prion protein molecules, as a consequence of ingestion gain entry to the brain and cause normal PrPs to take on the disease causing PrP^{sc} form.

Currently a labeled mouse monoclonal antibody 3F4 has been shown to bind prions with a sensitivity of binding of the antibody of 5 picograms per ml. This indicates that billions of prion protein molecules or greater would be necessary to be present to support detection of binding of the antibody to the aberrant prion. Furthermore, the assay is complex, requiring selective precipitation of PrP^{SC} by sodium phosphotungstate. This inability to more sensitively detect the presence of the prions in TSEs has hampered an understanding of the disease, attempts to determine if a cure scenario is feasible, and development of a vaccine.

What is needed are methods and compositions that will detect the prion protein in a large sample of plasma and concentrate and collect the normal and the aberrant forms of the prion in a small volume. Furthermore, methods are needed for diagnostic assays that will detect the presence of the aberrant prion protein with high levels of sensitivity in the presence of a large amount of non-specific or normal prion.

What is needed are methods and compositions that recognize the presence of very low numbers of infectious or other targets in an excess of non-specific, non-target or normal material. The target may be nucleic acid, such as DNA and RNA, cellular, or protein, in nature. Ideally, these methods and compositions comprise diagnostic technology that supports high levels of specificity and sensitivity in testing procedures. Preferred methods and compositions comprise diagnostic tests that are configured for early detection of the pathologic agent or other target in the sample by examining the DNA, RNA, cell, or soluble protein in solution, to detect the pathologic target earlier in the infection time-course.

### SUMMARY OF THE INVENTION

The present invention is directed to methods and compositions for detecting pathological conditions. In particular, the invention comprises methods and compositions using biological factors, such as complement components, for detecting pathological conditions. Particularly described are assays for non-specific target elimination that allow for detection of low copy number targets in a large field of nontarget material. Such assays comprise methods comprising CMSA and MACMSA, which preferably comprise detection of complement proteins and components. The assays of the presentinvention can be used for detection of changes in cellular molecules or nucleic acids that are part of disease states or infections, or can be used for detection of molecules in the environment.

Not only do the methods and compositions of the present invention comprise detection of nucleic acid and other molecular targets, but the methods and compositions of the present invention comprise diagnostics at supramolecular levels to confirm the presence of the pathologic or other cellular targets in tissues. The present invention comprises the analysis of only the cell subset of interest in a very large cell specimen and has the ability to compartmentalize and assay each cell component for the analyte of interest. Other embodiments comprise target analyte sorting and separation from non-specific analyte for increased sensitivity of detection. CMSA comprises the fixation and activation of complement by interactions between cell subset specific surface membrane proteins, and monoclonal or other antibodies. The initiation of the complement fixation process results in the production of the C3a peptide in quantities directly proportional to the extent of complement fixation.

One embodiment of CMSA, called MACMSA, comprises use of a soluble immunogen found in the cytoplasm or released into the cellular environment. These methods and compositions are used to diagnose the presence of pathologic or other specific soluble immunogens in the cytosol or those released into the surrounding media. The diagnostic assays of the present invention are able to accurately diagnose the presence of the disease state and also determine the position of the patient in the time-course of the disease or other process.

### DETAILED DESCRIPTION

The present invention comprises methods and compositions for the detection of low copy number targets of interest in diagnostic specimens in the presence of a large excess of normal material. The present invention can be used for diagnostic tests and has the capability to analyze specimens at the molecular, cellular, and tissue levels.

Methods and compositions of the present invention comprise non-specific target elimination, NTE. NTE detects targets and supports high limits of specificity and sensitivity. Embodiments of NTE include the Haystack Processing technologies such as TPA (Target Protection Assay), RFTA (Restriction Fragment Target Assay), EAD (Enzyme Assisted Diagnostics) and CPA (Cutter Probe Assays),and other compositions and methods as described in U.S. Patent numbers 5,962,225, and 6,100,040, and U.S. Patent Application Nos. 09/633,848; 09/992,128; 08/739,80; 09/569,504; 09/443,633; 09/702,066; 10/055732; 09/705,067; and other detection methods such as those disclosed in U.S. Patent Application Nos. 09/776,568; and 09/933307; each of which is incorporated herein in its entirety. The present invention is directed to methods and compositions including NTE which comprise Selective Target Monitoring technologies (STM) with Complement Mediated Signal Amplification (CMSA) and MACMSA (Membrane Associated Complement Mediated Signal Amplification).

Not only do the methods and compositions of the present invention comprise detection of nucleic acid and other molecular targets, but the methods and compositions of the present invention comprise diagnostics at supramolecular levels to confirm the presence of the pathologic or other cellular target in tissues. STM functions on a cellular or nuclear level to negate the presence of normal cells or nuclei in the sample by the analysis of only the cell subset of interest in a very large cell specimen and has the ability to compartmentalize and assay each cell component for the analyte of interest. These low copy number analytes are detected at low copy numbers by generating a signal from the specific analyte of interest, while no signal occurs from the normal or non-specific analytes present in the compartment. Other embodiments of STM comprise target analyte sorting and separation from non-specific analyte for increased sensitivity of detection. STM on a cellular level comprises CMSA. CMSA comprises the fixation and activation of complement by interactions between cell subset specific surface membrane proteins, and monoclonal or other antibodies. The initiation of the complement fixation process results in the production of the C3a peptide in quantities directly proportional to the extent of complement fixation.

CMSA is used for detection of target cells and supports NTE in any sample, particularly biological samples including, but not limited to, all body fluids, disaggregated cells, such as those derived from tissue samples, lymph nodes and fine needle aspirates, and environmental samples. An embodiment of CMSA analysis on a cellular level is taught in U.S. Patent Application 09/776,568, U.S. Provisional Nos. 60/218,460 and 60/226,825, all of which are incorporated herein in their entirety. For example, the intact cell, or cell membrane ghost, or nucleus is treated with a monoclonal antibody specific for a surface protein of interest, thereby forming an Ab/Ag complex that fixes complement. In the presence of all the complement components, complement is activated to produce C3a peptides, whose quantity is directly proportional to the number of target cells present. The target analyte comprises any cell subset, an HIV infected T-cell, a dysplastic cell, and a neoplastic cell or may also be a cell membrane or cell nucleus, as well as an immunogenic carcinogen or pathologic prion protein molecule.

C3a peptides are produced due to the interactive presence of a lipid membrane containing a unique surface protein (immunogen), a monoclonal or polyclonal antibody, and the complement cascade components. The presence and quantification of the C3a peptide produced may be achieved by any number of methods known to those skilled in the art and discussed herein or in related documents. The key to CMSA is the presence of a lipid membrane that functions to amplify production of the C3a peptide by the complement cascade components. The present invention contemplates the use of lipid membranes found within the sample or lipid membranes that are added to the sample.

The methods and compositions comprising Membrane Associated Complement Mediated Signal Amplification (MACMSA) are used to for sensitive soluble protein analysis. In an embodiment of this method, RBC sensitized stroma, comprising antibody to the unique protein attached to a RBC lipid membrane, interacts with the target analyte molecules present in the sample. Presence of the specific target analyte causes an Ag/Ab reaction to occur at the surface of the lipid RBC membrane, which in the presence of the complement components results in the full amplification of C3a peptide production and sensitive confirmation of the presence of the immunogenic target analyte. MACMSA is capable of molecular confirmation of a cellular diagnostic result as is taught in U.S. Patent Application 09/776,568, U.S. Provisional Nos. 60/218,460 and 60/226,825, all of which are incorporated herein in their entirety.

Soluble protein or peptide targets or other immunogenic molecules, whether pathologic or not, can be analyzed by STM on a soluble cytoplasmic molecular level that is monitored by use of MACMSA. MACMSA can also sensitively detect protein/peptide targets in any body fluid or other liquid sample. Another function of MACMSA is to detect and monitor non-protein chemicals in solution that are immunogenic thereby fixing and activating complement via the classical pathway, and to detect and monitor polysaccharides or other related molecules that fix and activate complement via the alternative pathway. MACMSA is used for detection of soluble target molecules in any biological or environmental fluid sample including, but not limited to, all body fluids, any soluble protein fluid suspension, environmental fluids, and chemical and material processing fluids containing the soluble, immunogenic target analyte.

Unique pathologic proteins or other immunogens at low molecule number in a vast excess of normal proteins are identified, using STM with high specificity and sensitivity. The specificity comes from the use of multiple specificity steps, and the sensitivity is supported by the minimization of signal background by non-specific target elimination in the fluid samples, either extracellular or intracellular, and generation of signal from all target molecules either intracellular or of exogenous target in a large sample of analyte.

### DESCRIPTION OF TABLES

Table I represents the stoichiometry of C3a peptide production by fixation and activation of complement by both the classical and alternate pathways, which mediates target signal amplification in the MACMSA complement fixation assay.

Table II presents the TSE time-course and its requirements with references.

Table III represents some of the interactions between plasminogen (PGₙ) and plasma proteins, catalogued to delineate the PGₙ role in the BSE/TSE infection time-course.

Table IV depicts some of the in vivo interactions and functions of plasminogen, catalogued to delineate the PGₙ function in the BSE/TSE infection time-course.

Table V is the Meta Screen Bovine Multiplex Diagnostic Assay, depicting the three MACMSA complement fixation assays.

Table VI is the Meta Screen Bovine Multiplex Diagnostic Assay, the molecular direct automated analysis of RNA.

### SELECTIVE TARGET MONITORING (STM)

STM cellular diagnostic technologies function on a cellular or nuclear membrane level to diagnose the presence of a pathologic or other cellular target, usually a cell or nuclear subset A preferred embodiment comprises use of CMSA methods for signal amplification for the sensitive detection of the pathologic cell or nucleus. CMSA is based upon the activation and fixation of complement by addition to the target cell of an antibody specific to a cell surface or nuclear membrane protein. In eucaryotic cells, the classical complement activation pathway is activated and the extent and target presence monitored by production of the C3a peptide. In prokaryotic cells, surface carbohydrates similarly participate by activation of the alternate complement fixation pathway also resulting in the production of the C3a peptide. One embodiment of CMSA, called MACMSA, comprises use of a soluble immunogen found in the cytoplasm or released into the cellular environment. These methods and compositions are used to diagnose the presence of pathologic or other specific soluble immunogens in the cytosol or those released into the surrounding media. The diagnostic assays of the present invention are able to accurately diagnose the presence of the disease state and also determine the position of the patient in the time-course of the disease or other process.

Signal amplification in STM on a cellular or nuclear level is directly proportional to the extent of complement fixation and activation. The cell surface membrane and nuclear membrane protein markers react with the specific monoclonal or other antibody to the immunogens resulting in fixation and activation of complement Also cell surface polysaccharides and other materials fix and activate complement via the alternative pathway. The extent of complement fixation may be monitored as a function of the number of C3a peptides produced upon activation of fixed complement molecules, known to those skilled in the art.

### MEMBRANE ASSISTED COMPLEMENT MEDIATED SIGNAL AMPLIFICATION (MACMSA) AND TARGET SIGNAL AMPLIFICATION

The methods and compositions comprising MACMSA comprise embodiments that function at the molecular level by using compositions comprising attachment of an antigenic epitope or a peptide comprised of numerous epitopes to an oligonucleotide that acts as a reporter probe in nucleic acid assays. One embodiment of MACMSA comprises using a single epitope to produce increased numbers of C3a molecules after binding of antibody sensitized RBC stroma to the epitope in the presence of complement followed by complement fixation and activation.

The extent of complement fixation and activation is influenced by many factors. These factors include avidity of the epitope and monoclonal antibody, and concentration of key intermediates in the complement cascade. For example, spiking native complement with additional C3 will increase the numbers of C3a produced by the presence of a single epitope in the assay. Other factors are determined by the method of complement fixation employed, either the classical or alternate pathway and the relative effect of C3 spiking on complement fixation by each; and the use of sensitized RBC stroma used to amplify the C3a production signal from a soluble immunogen, and methods of quantification of the resulting C3a peptide. The factors influencing C3a production in MACMSA, when optimized, can provide significant C3a peptide production per single target.

### MACMSA AND SINGLE TARGET PROTEIN DETECTION

To achieve the full signal amplification effect of a soluble protein target in STM, a preferred embodiment requires the introduction of a lipid membrane to the assay namely antibody sensitized red blood cell stroma.

### PRODUCTION OF SENSITIZED RBC STROMA

A preferred embodiment for production of RBC sensitized stroma employs the production of an IgG antibody pair, more preferably each IgG antibody has a different specificity. For example, one IgG of the pair is an IgG anti-Rh monoclonal antibody used to attach the antibody pair to the RBC surface, without any need for chemical modification of the RBC. The second IgG of the pair is an IgG anti-epitope monoclonal antibody used to bind the epitope present on the reporter probe and to fix and activate complement.

The red blood cells carrying the Rh determinants allow attachment of the antibody pair to the RBC membrane. A benefit of using the Rh determinant is that the Rh/antrRh complex is known to not fix complement. Any other Ag/Ab pair could also be employed in the methods and compositions of the present invention. RBCs with Ab pairs are referred to as sensitized.

In place of the anti D antibody attachment, any other site could be used that is expressed homogeneously on the RBC surface. In another embodiment an Fab or (Fab)2 fragment specific for the alternate attachment site may be used due to the absence of the Fc region on the attachment antibody fragment used.

The sensitized RBCs are washed and lysed in a hypotonic buffer solution and the resulting membrane material is referred to as stroma. Other embodiments call for the use of digitonin to produce a more intact RBC stroma or any method, which is known to those skilled in the art. The stroma is washed to remove RBC contents and resuspendedin a suitable buffer. The stroma may now be used as a reagent.

Addition of stroma, the reporter probe with epitope and fresh complement and cofactors allows maximal C3a production. The solution may now be assayed for C3a peptide production by use of any procedure known by those skilled in the art, such as ELISA and sensitized RBC lysis or any other method known by those skilled in the art.

### SIGNAL AMPLIFICATION OF SOLUBLE PROTEIN TARGETS IN MACMSA

In these embodiments of STM, complement fixation and activation is quantified by a novel method, namely detection of production of the inactive complement peptides (ICP), C3a. Detection of the ICPs, preferably C3a, is achieved by assays for proteins or peptides that are known to those skilled in the art, including but not limited to, competitive and sandwich immunoassays such as ELISA assays, immunoMTRF or assays included in the present invention such as complement mediated signal amplification (CMSA) and lysis of sensitized RBCs, and lysis of liposomes containing fluorescence and quencher molecules.

Complement is a group of at least 25 glycoproteins with varying electrophoretic mobilities. Most circulate in the blood in an inactive precursor form and have effects in the body only after activation. Two major functions of complement in vivo are to promote the inflammatory response and to alter biological membranes to cause direct cell lysis or enhanced susceptibility to phagocytosis. Cell lysis occurs when antibody-mediated complement is fixed and activated by sequential interaction of the entire complement cascade. Most of these interactions result in the cleavage of an inactive protein with the release of small peptides in the complement response. In vitro, these peptides have no function, or are called inactive complement peptides (ICPs). The peptides that do not participate in a direct complement response, meaning the lysis of cells or the opsonization of cells, are referred to herein as inactive complement peptides (ICPs). These inactive complement peptides (ICPs) have multiple in vivo functions: chemotaxis, enhancement of phagocytosis, alteration of vascular permeability, and stability of cell membranes (platelets and granulocytes). In a few instances, inactive proteins aggregate resulting in an active protein.

### THE CLASSICAL COMPLEMENT PATHWAY CASCADE:

The first complement component C1, attaches to the Fc portion of immunoglobulin molecules that have the appropriate binding site in the CH2 domain of the heavy chain. All mu (□) chains have this site, and most gamma (γ) chains. C1 is composed of 3 subunits: C1q, C1r, and C1s held together by calcium ions. If IgG is the type of antibody used, two adjacent protein antigenic sites must each bind an antibody molecule to form a doublet arrangement to provide the specific conformation for binding of the C1 complex. One IgM pentamer can bind the C1 complex. C1q binding to the FC region of the antigen/antibody complex undergoes a conformational change that activates C1r, which in turn activates C1s, and fixes complement.

The following represent the steps in complement fixation and activation resulting in the production of the ICPs (C2a, C4a, C3a, and C5a).

Each molecule of C1q bound or fixed to the target membrane will produce at least an equivalent number of C3 convertase molecules and the ICPs, C2a, C4a, C3a, and C5a. At least one C3 convertase molecule is formed per one C1q molecule initially bound. Thousands of surface membrane proteins are expressed on a single cell, thus activation of complement fixed by multiple sites on a single cell or nuclear membrane can produce thousands of C2a, C4a, C3a, and C5a ICPs.

C1s propagates the complement sequence by cleaving C4 into C4a and C4b and cleaving C2 to uncover a labile binding site. C4b contains a binding site and attaches to the cell membrane. C4a is released into the solution in vivo to stimulate anaphylaxis by stimulating mast cell degranulation and histamine release, thereby increasing vascular permeability. This released peptide may be used in the present invention to amplify the signal from a target.

C2 attaches to the C4b molecule on the cell membrane. The larger fragment C2b combines with C4b to produce C4b2b, called C3 convertase, which possesses enzymatic activity. Each initial C4b2b molecule (C3 convertase) can generate attachment of hundreds of additional C4b2b (C3 convertase) active complexes to the cell membrane in proximity to the C1q binding site (the lipid structure is a requirement for this event), and in doing so, releases additional C4a and C2a ICPs which can be used for signal amplification methods in the present invention.

The third step, also an amplification reaction, is based on the function of all the bound C3 convertase molecules (C4b2b) to each cleave hundreds of C3 molecules in solution resulting in release of additional C3a peptide fragments into the solution. This peptide has anaphylatoxin activity in vivo, and will be exploited as a signal amplification marker method in vitro. The C3b larger fragment binds to the cell membrane complex or decays in solution. C3b fragments by themselves are not active catalytically and do not promote cell lysis but do increase phagocytosis upon attachment to the cell (opsonin activity in vivo). The importance here is the additional production and release of C3a into the solution in vitro and plasma in vivo.

Some C3b molecules join the extensive numbers of C3 convertase attached to the entire cell membrane forming C4b2b3b5b or C5 convertase releasing the C5a ICP into the solution.

In the presence of C5b, molecules of C6, C7, and C8 and a variable number of C9 molecules, assemble themselves into aggregates in the presence of Zn+2 called the membrane attack complex (MAC). The complex compromises the integrity of the cell membrane by altering permeability of the membrane and results in cell lysis.

It must be noted that C3a generation in CMSA and MACMSA is only one embodiment of a complement fixation assay, wherein C3a is the measure of the extent of complement fixation and activation. In related applications, it has been stated that any other peptide produced or any other complex MAC complex formation, etc., characteristically formed during complement fixation and activation can be used as a measure of complement fixation and activation. This is relevant to any produced stable product or stable complex formed by the classical pathway as well as the alternate pathway during complement fixation and activation.

### THE ALTERNATE PATHWAY COMPLEMENT CASCADE

Cleavage of C3 and subsequent activation of the remainder of the complement cascade occurs independently of complement fixing antibodies. Cell surface particulate polysaccharide and lipopolysaccharide molecules, endotoxin, trypsin-like enzymes, and Ag/Ab complexes of IgA, and IgG4, that do not activate C1, all function to activate the alternate pathway. The activation is mediated by the cleavage of C3 into C3a which is released in solution and C3b. This molecule would be rapidly degraded in the fluid phase (classical pathway), but in the alternate pathway, C3b becomes stabilized by binding to the surface of a particulate activator of the alternate pathway called factor B, forming a stable C3b-factor B complex, itself interacting with a serum protease (factor D), cleaving factor B to produce C3bBb, that functions as a C3 convertase, again catalytically producing many additional C3a peptides.

The alternate complement activation pathway is activated by all viruses, bacteria, yeast or any other microbe containing polysaccharide or lipopolysaccharide elements in its exterior cell wall.

One embodiment of the present invention, the novel in vitro use of the complement cascade and the generation of the ICPs in the amplification of a signal to detect very low copy number of targets, is described herein.

### SIGNAL AMPLIFICATION IN STM

The present invention comprises novel and sensitive methods for signal amplification, called CMSA and MACMSA. Activation of the complement cascade results in the production of millions of inactive complement peptides (ICPs). Analysis of the sample for the detection and quantification of the ICPs results in the generation of 40 million ICPs per pathologic cell membrane, or nucleus (CSMA), and generation of 40,000 ICPs per soluble protein target or immunogenic epitope with the involvement of complement fixing Ag/Ab reactions in proximity to a lipid matrix (MACMSA).

Table I summaries the production of the ICPs and theoretical quantification provided by CMSA.

**TABLE I**

| **ICP Characterization and Quantification in CMSA** | | |
|---|---|---|
| **10³ sites to fix C' per membrane surface** (First Amplification Step) | **Nature of ICP generated** | **Number ICPs produced based on binding of each C1s molecule** |
| C1q, C1r, C1s | NONE | NONE |
| C4b | C4a | 10³/cell or nuclear membrane |
| C4b2b3b | C3a | 10³/cell or nuclear membrane |
| C4b2b3b5b | C5a | 10³/cell or nuclear membrane |
| C6,7,8,9 | NONE | NONE |
| (Second Amplification Step) | **200 fold increase in ICPs** | |
| C1q, C1r, C1s | NONE | NONE |
| C4b | C4a | 200 x 10³ 2x10⁵ per C1s bound |
| C4b2b3b | C3a | 2 x 10⁵ |
| C4b2b3b5b | C5a | 2 x 10⁵ |
| C6,7,8,9 | NONE | NONE |
| (Third Amplification Step) | **200 fold increase in ICPs** | |
| C1q, C1r, C1s | NONE | NONE |
| C4b | NONE | NONE |
| C4b2b3b | C3a | 200 x 2 x 105 or 4 x 10⁷ or 40 Million (theoretical) |
| C4b2b3b5b | C5a | Not amplified here (same value as amplification step number 2) NONE |
| C6,7,8,9 | NONE | NONE |
| **SUMMARY**: ICP numbers produced in CMSA based on the presence of a single pathologic target cell | | |
| | • Primary Amplification Step 3 x 10³ ICPs | |
| | • Secondary Amplification Step 6 x 10⁵ ICPs | |
| | • Tertiary Amplification Step 4 x 10⁷ ICPs | |
| Total ICPs generated per single pathologic target 4.0603 x 10⁷ ICPs 40 Million | | |

It is theorized that the above results are achieved by reducing the ICP generation 1000-fold, due to the ability of a single immunogenic epitope to fix 1000-fold less complement than an average cell membrane.

A preferred ICP is the peptide fragment C3a, because it is found in very high numbers after complement fixation. Production of other ICPs (C4a, C2a, and C5a) may also be detected although they provide less than one percent of the total signal generated by the detection of a single pathologic cell, nucleus, or nucleic acid species.

In general, the novel in vitro use of the complement cascade to quantify the presence of a pathologic cell or nucleus is based upon monitoring the extent of complement fixation and activation as a function of the number of inactive complement peptides (ICPs) that are produced. Basically, each target cell fixes thousand of complement molecules after addition of antibodies specific for the target cell surface protein and the subsequent reaction with the complement cascade. The initial complement molecules that are fixed can themselves exert an additional 200-fold amplification effect. These complement molecules also provide for another 200-fold signal amplification effect later in the course of the complement cascade. This results in the following theoretical total signal amplification profile in CMSA
a) Multiple cell surface protein markers on the dysplastic cell each fixing complement, yielding 1000-fold amplification per pathologic target,
b) Primary 200-fold amplification during early stages of complement fixation,
c) Secondary 200-fold amplification at a later step in the complement cascade. Total 40 million ICPs produced per target.

In MACMSA, the following represents the total signal amplification profile:
a) A single soluble protein or reporter immunogenic epitope fixes one complement molecule.
b) Primary 200-fold amplification effect during early stages of complement fixation that is lipid membrane dependent requiring the use of the RBC sensitized stroma reagent
c) Secondary 200-fold amplification at a later step in the complement cascade (membrane independent). Total 40,000 ICPs produced per target.

### SIGNAL AMPLIFICATION

Methods of signal amplification using the classical complement pathway employ methods of CMSCA and MACMSA. Signal amplification methods for the alternate pathway is similarly initiated by a step wherein a thioester on native C3 binds to polysaccharide, such as a polysaccharide on the surface of an organism. Next, the binding of Factor B and its subsequent activation stabilizes the complex:
C3H₂O + Factor B + Factor D = C3bBb + C3a
C3bBb = activated Factor B or C3 convertase

The first signal amplification step occurs by the convertase cleaving numerous native C3 molecules producing numerous C3a peptides and additional C3b molecules that attach to the complex to form additional C3 convertase, that release additional C3a in the solution.

The C3 convertase (C3bBb) cleaves hundreds of C3 molecules generating additional C3b molecules, which attach to the complex and amplifies its activity. Cleavage of the C3 mediates release of hundreds of C3a ICP molecules to mediate amplification in vivo of the immune response and in vitro signal amplification.

The second level of signal amplification employs the aggregation on the surface of a microorganism or a protein aggregate of numerous C3b units, Factor B, and Properdin (stabilizing protein) acts as a potent C5 convertase producing hundreds of C5a (ICPs), thus cleaving C5 to an active C5b and release of a C5a into the solution. The remainder of the complement cascade is identical to later steps in the classical pathway. Thus, the ICPs, generated by complement fixation of the classical complement pathway, or the alternate complement pathway are used for in vitro signal amplification target detection strategies.

### STOCHIOMETRY OF ICP PRODUCTION VIA THE CLASSICAL COMPLEMENT PATHWAY (See Table I)

In Table I, it is represented that three levels of C3a production or signal amplification occurs based upon CMSA treatment of a cell or nuclear membrane.

First, thousands of surface protein molecules on a single cell or a single nuclear membrane fix thousands of C1 molecules producing a minimum of thousands of C3a peptides post complement fixation.

Second, there exists a 200-fold amplification per each of the C1a molecules fixed due to the presence of the membrane proximity component for complement activation.

Third, there exists an additional 200-fold amplification per each of the 2 x 10⁵ bound C3 convertase (C4b2b3b) molecules by cleavage of additional solution C3 and formation of additional C3a peptide for a total of 4 x 10⁷ or 40 million molecules of C3a peptide generated per target membrane.

### THEORETICAL STOCHIOMETRY OF ICP PRODUCTION VIA THE ALTERNATE COMPLEMENT PATHWAY

C3a production by the alternate pathway must be empirically determined. Similar signal amplification quantification can be configured based on the cyclic C3 convertase enzymatic complexes formed. Though not wishing to be bound by any particular theory, it is believed that the absolute numbers of ICPs produced in the alternate pathway are on the same order of magnitude or greater than that observed by complement fixation and activation via the classical pathway.

### DETECTION AND QUANTIFICATION ASSAYS FOR THE ICPS

### (C4a, C2a, C3a, C5a)

Many assay strategies are available to determine the presence and quantification of the individual or combined ICPs. The present invention comprises assays for measuring the presence and number of individual or combined ICPs and is not limited to the assays and embodiments disclosed herein. The individual ICPs can be quantified by assays for proteins, including but not limited to sandwich ELISA assays, or similar assays that use a capture antibody bound to a solid support and a different labeled reporter antibody both specific for different epitopes on each ICP (C4a, C2a, C3a, C5a).

For example, an embodiment of the C3a sandwich ELISA assay is configured using a biotinylated anti-C3a reporter antibody and is followed by addition of an IgG anti-biotin alkaline phosphatase polymer conjugate to facilitate signal generation per C3a molecule by introduction of the substrate, 1,2-dioxetanes. Any other enzyme known to those skilled in the art may be used to quantify the number of C3a molecules. The enzyme may provide a color signal, a fluorescent signal, or a chemiluminescent signal, all known to those skilled in the art.

A preferred embodiment of the signal generated by the C3a peptide molecules is mediated by the use of an anti-biotin alkaline phosphatase polymer, known to generate 4 logs of signal per polymer molecule. The polymer is then reacted with a chemiluminescent substrate generating a stable light signal. One such substrate is the 1,2-Dioxetanes, which have been shown to detect 0.01 attomole quantities of alkaline phosphatase enzyme, translating to a ten-fold increased level of target detection by the enzyme polymer. This detection system will support unprecedented high levels of target detection and, due to the nature of antibody conjugates to enzymes, will provide a relatively low background in the negative controls

Such methods may also be automated. An example is shown below.
Step I. Prepare a magnetic bead with a covalently bound IgG anti-C3a capture antibody. The binding can be achieved by any chemistry known to those skilled in the art such as covalently linking a carboxylated magnetic bead to the primary amine on the n-terminal end of the antibody molecule, or any other chemistry known to those skilled in the art.
Step II. The magnetic bead is washed to remove non-bound capture probes and
Step III. Conjugated beads are added to a sample containing the C3a peptide in solution, which is mixed and incubated.
Step IV. The magnetic beads are washed to remove non-specific bound materials
Step V. Addition of another antibody, IgG anti-C3a, which has reporter function and is specific for a different epitope on the C3a peptide molecule. This antibody possesses an alkaline phosphatase (AP) polymer covalently attached to it. This may be generated by any method known to those skilled in the art, the preferred one being attachment antibody N-terminal amine of the maleimide derivative of the AP polymer, which results in covalent bond formation. Any other chemistry may also be employed.
Step VI. Wash to remove unbound reporter probe. The number of washes and the wash buffer may be critical in resolving non-specific signal from unbound reporter enzyme.
Step VII. Addition of the magnetic beads to a solution containing the 1,2-Dioxetane substrate and incubate under conditions for the production of a stable chemiluminescent signal.

The reporter antibody, and hence the target, is detected by the activation of a chemiluminescent substrate to produce light by enzymatic catalysis.

The reporter antibody can also be detected using immuno MTRF methods as disclosed in U.S. Patent Application No. 09/443,633 or by conjugating a label, such as a single molecule of fluoroscein isothiocyanate, to each ICP reporter antibody.

Another method of the present invention for C3a quantification comprises steps to identify and quantify the specific ICP of interest using sensitized RBCs conjugated with anti-specific ICP antibodies, which will only react with the free-floating ICPs in solution. In this embodiment RBCs linked to anti-ICP monoclonal antibodies will in the presence of complement undergo complement-mediated immunoerythrocyte lysis, releasing hemoglobin for quantitation.

The extent of RBC lysis is directly proportional to the quantity of ICPs produced and targets present.

Another method for assay of C3a production would be the use of IgG anti-C3a antibody imbedded on the surface of a liposome containing fluorescence and quencher molecules in close proximity, so that no fluorescent signal can be detected. Introduction of a C3a peptide to the antibody-sensitized liposome, in the presence of the complement components will result in complement mediated lysis of the liposome, releasing the fluorescence and quencher molecules into the solution. Their release and separation can be monitored by the detection of a fluorescent signal. The extent of liposome lysis is directly proportional to the quantity of ICPs produced and targets present.

### GENERATION OF SENSITIZED RBCs FOR C3a ASSAY: RBC ENZYME TREATMENTS

One embodiment of the present invention comprises methods to identify and quantify specific ICPs of interest comprising use of sensitized RBCs that are conjugated with specific anti-ICP antibodies that will only react with the free-floating ICPs in solution and in the presence of fresh complement, result in red blood cell lysis upon binding of free ICPs with subsequent complement fixation and red blood cell lysis.

The sensitized or immuno RBCs can be generated by stripping the RBCs with a proteolytic enzyme such as bromelain, ficin, or papain and by other methods known to those skilled in the art, that attach the ICP specific antibodies to the RBC surface, producing sensitized immunoerythrocytes which bind the free floating ICP in solution. This attachment of an antibody to the stripped RBC surface by simple exposure of the antibody to the erythrocyte provides a non-covalent attachment of the antibody molecule, and is sufficient for some applications. Due to the fact that chemical modification of the RBC surface involves increased fragility of the modified RBC, which may result in the spontaneous release of hemoglobin and make quantification of the ICP peptides difficult, other methods are also contemplated by the present invention.

A novel process for production of antibody sensitized RBCs is mediated by the use of an IgG antibody pair. The characterization of the molecule is as follows:
1. Two IgG molecules are attached to each other by any method known to those skilled in the art, where the attachment does not interfere with the antibody binding sites.
2. One antibody must be specific to any of the ICP peptides for assay; for example, the IgG anti-C3a antibody used in the C3a peptide assay. Other embodiments require this antibody to be specific for any immunogenic epitope on the target.
3. The other antibody is specific for an antigen on the RBC. A most preferred embodiment comprises use of an antibody specific for the Rh determinant. The Rh determinant extensively covers the RBC membrane with thousands of molecules and this is the site at which the antibody pair binds to the erythrocyte. This antigen/antibody reaction does not fix complement. This is important in light of the use of this immunoerythrocyte in the presence of fresh complement to monitor attachment of the C3a peptide to the complement fixing anti-C3a antibody in close proximity to the RBC surface. Any interactive antigen/antibody interaction that does not fix complement may also be employed.
4. The Rh determinants on the RBC surface are responsible for bringing the antibody to the C3a and other peptides in close proximity to the lipid membrane surface without altering the stability of the immunoerythrocyte.

The sensitized immunoerythrocyte in the presence of the corresponding peptide and fresh complement will undergo lysis by the membrane attach complex and hemoglobin will be released.

### THE ANTIBODY PAIR METHOD FOR IN VIVO NEUTRALIZATION OF A PATHOLOGIC ANALYTE BY SENSITIZED RBCs

Another embodiment for use of the antibody-pair molecule may involve its use in vivo to neutralize the activity of a pathologic analyte. This analyte may be a viral particle, antibody molecule, dysplastic or cancer cell, and even an immunogenic environmental carcinogen. Attachment of the IgG anti Rh-IgG anti pathologic analyte antibody pair to the RBC surface would facilitate the immediate attachment and neutralization of the pathologic analyte to any of the RBCs that have been sensitized.

Neutralization of the activity of the pathologic analyte would immediately block its reactive effect and would initiate its removal from the body mediated by macrophage phagocytosis or the function of another clearance system in the spleen and liver. It is known to those skilled in the art that RBCs possessing immune complexes on their surface are rapidly cleared by these body systems.

### PRODUCTION OF SENSITIZED RBC STROMA FOR USE IN MACMSA

MACMSA requires the interaction of a lipid/antibody complex with a soluble protein or reporter probe immunogenic epitope. The preferred embodiment for production of this complex is the sensitization of the RBCs by the aforementioned method with subsequent lysis of the sensitized RBCs in a hypotonic buffer solution resulting in the production of antibody attached lipid membrane (RBC stroma) that will exert the full signal amplification effect of the immunogenic epitope or soluble protein by the MACMSA process. Stroma production is achieved by placement of the immunoerythrocytes in a hypotonic buffer resulting in RBC lysis and membrane ghost formation. The stroma is then washed in buffer and resuspended in buffer for use as a reagent. Any other method known to those skilled in the art may be used.

### STM/PRION SORTING IN SOLUBLE PROTEIN SAMPLES

One embodiment of prion sorting in a protein sample can be achieved by attachment of a monoclonal antibody at its C-terminal end to a magnetic bead. The magnetic beads are placed in a large volume solution such as plasma or brain biopsy extract or any other and mixed. The epitopes available for interaction on the C-terminal end of the prion molecule remain exposed in both the normal and aberrant prion molecule.

The magnetic beads are collected with a magnet and washed in buffer. All the prion present in the sample will be separated from all other soluble proteins. The C-terminal antibody will capture the normal and pathologic prions due to the continued accessibility of the epitopes of both forms even after the transition has occurred.

In this embodiment the prion sorted magnetic bead is treated with a monoclonal antibody available to the N-terminal end of the prion molecule (the β sheet isoform end) that is labeled with an alkaline phosphatase polymer or any label known to those skilled in the art. Both are incubated and washed in buffer.

The N-terminal end of the pathologic prion has undergone a transition from an α to a β sheet form. During this transition, epitopes, normally found on the N-terminal end are covered and new epitopes exposed. To date no monoclonal antibodies have been found that successfully discriminate the α sheet form (normal prion) from the β sheet form of the aberrant prion.

Monoclonal antibodies specific for the N-terminal end of the pathologic prion are necessary for use in these sensitive diagnostic assay embodiments.

Next, the magnetic beads are exposed to a chemiluminescent substrate, such as the 1,2-Dioxetanes, which would be able to detect 0.01 attomoles quantities of alkaline phosphatase enzyme. Theoretically, supporting increased sensitivity than that achieved by prion precipitation by sodium phosphotungstate and time resolved fluorescence previously mentioned.

### CURRENT PrP^{sc} DIAGNOSTIC ASSAY: PROBLEMS WITH SPECIFICITY AND SENSITIVITY

To date there has only been one report of a monoclonal antibody specific for the aberrant prion that would not react with normal prion. This report was non-substantiated resulting in the following strategy for post-mortem brain assay.

First, since one could not differentiate between the normal and aberrant prions, one needed to rely on the protease (proteinase K) resistance of PrP^{SC} post enzyme treatment, not observed with PrP^{c} or PrP enzyme treatment.

Once the post mortem sample is treated by proteinase K then the surviving prions are reacted with the non-discriminatory prion monoclonal antibodies currently used. The rationale is that the only surviving prion must be an aberrant or β sheet conformation prion. It is known to those skilled in the diagnostic assay design that this type of assay is fraught with sensitivity problems, and that the extensive sample manipulations, and improper protease treatment could also result in specificity problems.

### STM MACMSA SENSITIVE DETECTION OF PATHOLOGIC PRIONS STRATEGY FOR A BLOOD BASED EARLY DIAGNOSTIC FOR BSE

Current post-mortem brain diagnostic assays currently are based upon selective destruction of normal prion in a sample by protease and guanidinium treatment followed by ELISA assay for identification of the resistant prion, which is assumed to be the PrP^{sc} aberrant form. Also, Western blot has been shown to be of value in this area. The antibody in this assay recognized both normal and aberrant prion with no ability to discriminate between either. The success of these assays can be attributed to their insensitivity, namely, the lack of specificity of the anti prion antibody and the insensitivity of the Western blot.

For the post-mortem diagnostic assay to function, large samples of obviously diseased brain must be obtained, which will assure a positive result even with the use of such insensitive methods. Furthermore, these diagnostics are costly and time consuming.

What is needed is a more sensitive and rapid diagnostic for the post-mortem assay. Consideration that this assay would only provide disease (BSE) confirmation as a cause of death, an even greater challenge in diagnostic process design would be to develop a sensitive, rapid, and inexpensive early infection diagnostic assay for BSE and other TSEs.

It is known to those skilled in the art that early in the infection, blood plasma levels contain 10 to 1000 aberrant prions (PrP^{SC}) per milliliter (closer to the former earliest in the infection time-course). These early BSE assays could take the form of blood or other body fluid assays where a sample can be acquired by a non-invasive (urine sample acquisition) or a minimally invasive (blood draw) technique, or any other sterile sample taken for diagnostic purposes known to those skilled in the art.

Remembering that no antibody currently exists that exclusively and selectively discriminates the normal α sheet from the abnormal β sheet prion conformation poses difficulties in early diagnostic disease assay design.

The approach used in this embodiment of the invention was developed by thorough analysis of all the facts related to BSE and other TSE diseases, such as scrapies, where TSE animal model systems abound.

It is known to those skilled in the art that scrapies infection can be blocked by inhibition of B cell formation, T cell formation, and complement depletion (C1q deficient host). The disease inhibition can be reversed by reestablishment of T and B cell production and complement activity (reversal of C1q deficiency).

Table II presents a number of conclusions drawn relative to the TSE time-course and its requirements (appropriate references are indicated).

### SOME CONCLUSIONS REGARDING TSEs

**TABLE II**

| **Remark** | **Reference** |
|---|---|
| Replication of infectivity occurs in lymphoid organs | Eklund, et al. 1967 |
| Infectivity found in components of the lymphoreticular system (LRS), lymph nodes and Peyer's plaques in the small intestine where replication of infectivity can be demonstrated almost immediately following oral administration of prion preparations. | Aguzzi, A., et al. 1998 |
| Follicular dendritic cells (FDC) may be the main population of cells involved in LRS replication of prion. | Aguzzi, A., et al. 1998 |
| All mutations that disrupted the differentiation and response of B-cells prevented the development of clinical disease. | Aguzzi, A., et al. 1998 |
| Spongiform encephalopathy developed after peripheral inoculation in mice expressing immunoglobulin that was exclusively of the M subclass and without detectable specificity for the normal form of the prion PrP^{c} in mice with B-cells but no functional FDC. | Aguzzi, A., et al. 1998 |
| Differentiated B-cells are crucial for neuroinvasion by spongiform encephalopthy regardless of the specificity of their receptors. | Aguzzi, A., et al. 1998 |
| If prions were delivered to the CNS spongiform encephalopthy pathogenesis and prion expansion in the brain proceeded without any detectable influence of the immune status of the host | Aguzzi, A., et al. 1998 |
| B-cells may transport prions from lymphoid organs to nervous tissue | Aguzzi, A., et al. 1998 |
| The apparent protection of B-cell deficient mice from prion administered intraperitoneally may result in the absence of immunoglobulins. | Aguzzi, A., et al. 1998 |
| Complexing of PrP^{SC} with antibodies may favor nucleation (a process proposed to underlie the formation of prion infectivity) | Eigen, M. 1996 |
| Complexing of PrP^{SC} with antibodies may opsonized PrP^{SC} and enhance access to lymphoid sites of abnormal prion expansion | Aguzzi, A., et al. 1998 |
| One cannot exclude the possibility that IgMs below the threshold of detectability, or indirect effects of antibodies may be involved in spongiform encephalopathy pathogenesis. | Aguzzi, A., et al. 1998 |
| The TSE infected B-cells carrying PrP^{SC} are the bottleneck of disease promulgation. | Aguzzi, A., et al. 1998 |
| Interaction exists between the B-cells (carriers of infectivity) and the T-cells (unable to propagate infectivity). Spleen mice contain both T-cells and B-cells, and upon infection of the B-cells, a B-cell mediated secondary infection of the spleen mice's T-cells takes place. | Aguzzi, A., et al. 1998 |
| TSE infected B-cells are predictive of the pathological outcome and progression of disease | Aguzzi, A., et al. 1998 |
| In mouse scrapie, depletion of B-lymphocytes prevents neuropathogenesis after intraperitoneal inoculation probably due to impaired lymphotoxin-dependent maturation of FDCs, which are a major extracerebral prion reservoir. | Klein, M.A. et al. 1997; Klein, M.A. et al. 1998 |
| FDCs trap immune complexes with Fcγ receptors and C3d/C4b-opsonized antigens with CD21/CD35 complement receptors. | Klein, M.A. et al. 1997; Klein, M.A. et al. 1998 |
| Depletion of circulating immunoglobulins or of individual Fcγ receptors had no effect on scrapie pathogenesis if B-cell maturation was unaffected | Klein, M.A. et al. 1997; Klein, M.A. et al. 1998 |
| Mice deficient in C3, C1q, Bf/C2 and combinations thereof or complement receptors were partially or fully protected against spongiform encephalopathy i.p.upon exposure to limiting amounts of prions. | Botto, M., et al. 1998; Kuhn, S.E., Taylor, R.P., et al. 1998; Molina, H., et al. 1996 |
| Splenic accumulation of prion infectivity in complement deficient mice and PrP^{sc} accumulation was delayed, indicating that activation of specific complement components is involved in the initial trapping ofprions in lymphoreticular organs early after infection | Klein, M.A. et al. 1997; Klein, M.A. et al. 1998 |
| Lymphotoxin beta (LT beta) aids FDC in neuroinvasion | Montrasio, F.et al.2000 |
| Sympathetic nerve fibers transport the rogue prions through the peripheral nervous system into the CNS | Aguzzi, A., et al. 1998 |
| Plasminogen has a neurotrophic factor-like effect on cultured neurons | Nakajima, K.,et al. 1994 |

### CONSENSUS OF OPINION ON EARLY TSE TIMECOURSE EVENTS

It is, herein, proposed that these documented observations can be interpreted as follows. This predicted chronology of events that define the early stages of disease will support the presented early specific and sensitive diagnostic processes to bedetailed herein.

The T cells may function in antigen (aberrant prion) display, in order for B cell production of a weak initial antibody response (IgM initially). Complexation of the anti-prion antibody and the aberrant prion, will fix and activate plasma complement thereby depositing C3b and other complement fragments on the aberrant prion antibody complex. The C3b may then interact with the M cell (in intestinal Peyer's patches of man) surface C3b or other complement receptor, resulting in ingestion via phagocytosis of the Ag/Ab C3b complex. The infection of BSE by ingestion of tainted meat supports the intestinal site of entry into the body. Furthermore, prior to phagocytosis, an exposed ε lysine on the fold of the β sheet aberrant prion binds a serum plasminogen molecule at its Kringle 1-3 domain (Fischer, 2000). The role of this bound plasminogen is not well understood however; plasminogen has been definitively implicated in a number of documented activities in vivo.

Table III represents some of the documented interactions between plasminogen and plasma proteins. It is herein stated that the plasminogen Kringles, regions may bind n or c terminal lysine molecules as well as ε lysine residues present in the internal regions of the peptide/protein when accessible to the Kringle region.

**TABLE III**

| BIOLOGICALLY RELEVANT PLASMINOGEN (PLASMIN)- PROTEIN INTERACTION INVOLVES ANCHORING OF THE ZYMOGEN THROUGH LYSINE BINDING SITES IN THE KRINGLE DOMAINS. | | |
|---|---|---|
| **PGₙ or Plasmin binding to:** | **Function** | **Reference** |
| C terminal Lysine of fibrinogen involved: | Stimulate and enhance clot lysis | Christensen, U. 1985 |
| C terminal lysine residues of PGn and plasmin treated clots | Inhibition of plasmin | Hortin, G.L., et al, 1988 |
| Other proteins with C terminal lysine | Binding to α-enolase | Hamanoue,M, et al, 1994 |
| Other proteins with C terminal lysine | Inhibition of PGₙ | Sugiyama, N., et al, 1988 |
| Internal Lysine involved: | | |
| ε-Lysine of PrP^{SC} | Undetermined | Fischer, M.B., et al, 2000 |
| ε-aminocaproic acid | Inhibition of PGₙ | Sugiyama, N., et al, 1988 |

To understand the possible function of the plasminogen on the PrP^{SC} molecule, Table IV depicts some of the known documented in vivo interactions and functions of plasminogen, catalogued to delineate the PGₙ function in theBSE/TSE infection time-course.

**TABLE IV**

| PLASMINOGEN/PLASMIN FUNCTIONS | | |
|---|---|---|
| **Function** | **Example** | **Reference** |
| Thrombolytic activity | Clot lysis | Miyato, T. 1982 |
| Cell movement | Macrophage invasion in inflammation | Unkeless, J.C. 1973 |
| | Mammary cell involution after lactation | Ossowski, L. 1979 |
| | Breakdown of follicular wall for ovulation | Reich, R. 1985 |
| | Trophoblast invasion into endometrium during embryogenesis | Strickland, S. 1976 |
| | Angiogenesis | Gross, J.L. 1983 |
| | Keratinocyte accumulation after wound healing | Morioka, S. 1991 |
| Mediator in pathologic process Tumor of cell migration | cell growth, invasion of surrounding tissues, metastasis | Ossowski L. & Reich, E. 1983 |
| | Ability of cell bound plasmin to directly degrade extracellular matrix proteins: | Mullin D.E. & Rohrlich, S.T. 1983 |
| | Proteoglycans | Edmonds-Act, X. 1980 |
| | Fibronectin | Jilek, F. 1977 |
| | Laminin | Schlechte, W. 1989 |
| | Type IV collagen | MacKay, A.R. 1990 |
| | Indirectly responsible for lysis of matrix proteis through activation of metalloprotease proenzymes | Stricklin, G.P. 1977 |
| Activation of transforming growth factor β₁(TGFf1) to its active form | Regulation of inflammation | Khalil, N. 1996 |
| Extracellular or pericellular protease causing proenzyme conversion | Classic complement pathway | Agostoni, A.1994 |
| | Alternate complement pathway | Brade, V. 1974 |
| | Contact phase of blood coagulation | Cochrane,C.G. 1974 |
| | Proinsulin-insulin conversion | Virgi,M.A.G. 1980 |
| | Bradykinin generation from kininogen | Habal, F.M. 1976 |
| Proteolytic activation/destruction of activation/destruction of plasma proteins | γ-globulin | Janeway, C.A. 1968 |
| | Coagulation factor V | Lee C.D. & Mann, K.G. 1989 |
| | Coagulation factor VII | Denson K.W. & Redman, C.W. 1980 |
| | Anticoagulation protein C | Varadi, K. 1994 |
| Neuronal excitotoxicity | Binding of PGₙ to neuronal cells | Hamanoue, M.1994 |
| Interaction in vivo with plasma platelets | Normal prion PrP release in vivo | |

Plasminogen functions presented herein, pose a strong case for the requirement of plasminogen on the PrP^{SC} molecule for the invasiveness of the prion in early stage disease. This includes normal prion accumulation and invasion of the aberrant prion into tissues necessary at some stage of the infectious PrP^{sc} time-course.

PrP^{sc} entry at the site of the M Cell in the intestinal Peyers patches in man is supported by 1) an understanding of mucosal immunology (Mucosal Immunology, 1999), which is the effector site for vaccines in the body, 2) acceptance of the PrP^{sc} infection requirement for active B cells and T cells, and 3) the presence of the C3b complement receptor on the M Cell macrophage (fixed monocyte) surface.

It is not to be interpreted that this is the only mechanism of PrP^{SC} entry into the body, but only one of a number of mechanisms, some of which involve non-specific pinocytosis of digestive fluid by intestinal epithelial cells (IEC), uptake by the FDCs, uptake by B-cells, and other purported mechanisms.

Upon formation of the PrP^{SC}/PGₙ/antibody/C3b the PrP^{sc} complex enters the lymphatic system by some method, where it travels to all lymphoid organs including the tonsils and ultimately the brain. Movement of the PrP^{sc} complex in the lymphatic system may afford the complex protection from phagocytosis by the C3b receptors present on numerous phagocytic cell surface in phagocytic departments that are not involved in the infection and may even limit the infection by disintegrating the PrP^{sc} complex. Remembering that C3b receptors are found on all polymorphonuclear granulocytic macrophages in the circulation and all fixed monocytic macrophages in the body, some protective effect would be required.

An alternate sequence of events for PrP^{SC} infection could entail PrP^{SC} entry to the blood, post ingestion, followed by complex formation (host IgM antibody/primary antibody response, complement, and plasminogen addition), and transport to the brain, whereupon glial fixed monocytic cells possessing C3b receptors could phagocytize and internalize the PrP^{SC} complex and further the disease course.

Whatever events occur during the early staging of the BSE infection, the existence of the PrP^{sc} complex as presented herein is indicated.

### TARGETS FOR EARLY PrP^{SC} DETECTION

Peripheral infection, in particular, oral uptake of TSE infected material is epidemiologically most relevant for BSE, Sheep Scrapie, Kuru, and ₙᵥCJD.

Acceptance of this rationale provides a number of targets that may be of value in an early disease time-course, blood based pre-mortem PrP^{SC} assay. They are:
1. Presence of plasminogen on the β sheet conformation, PrP^{SC}
2. Presence of a host primary response antibody (IgM) or other antibody that may be found to bind to the PrP^{SC}
3. Presence of C3b or other complement cascade products such as C3d and C4b or other complement fragments on the PrP^{SC} molecule
4. Presence of PrP^{SC}, only possible for use as a target if a monoclonal antibody is produced that will discriminate PrP^{SC} from PrPc or PrP (not as yet known to be in existence).

### CHARACTERISTICS OF AN EARLY BLOOD DIAGNOSTIC ASSAY FOR THE PRESENCE OF THE ABERRANT PRION PROTEIN, PrP^{sc}

It is preferable that early diagnostic assays for a disease be highly specific, highly sensitive and capable of automation, as well as cost efficient, to assess the state of the animal or human, as well as for monitoring residual disease, once a treatment or cure is available. The high specificity of the assay, defined as the lack of false positive results, can be provided by a multi-step procedure that is designed to limit the generation of signal by non-infectious target analyte (prions).

The high sensitivity of the assay, defined as the lack of false negative results, can be provided by the ability to screen large amounts of sample analyte, namely prion from a large volume of plasma, for the aberrant prion.

Ease of use of the assay, such as by automation, allows for widespread use in limiting epidemics by monitoring the spread of disease and maintaining surveillance of the state of the animal or human. Furthermore, combined manual and automated assays are readily achieved with the ability to screen volumes of plasma sample.

In related applications, the concept of non-specific target elimination (NTE) was presented. Basically, NTE are process designs that limit the generation of signal or abolish the generation of signal from non-specific targets. On a molecular level the Haystack Processing inventions TPA and RFTA, previously presented, achieve such by the use of enzymes to degrade non-specific targets resulting in the generation of signal exclusively from the target analyte. These diagnostic processes function to detect very low copy number DNA and RNA targets. On a supramolecular level (whole cells, intracellular and extracellar proteins, and other immunogens), the Haystack Processing invention Selective Target Monitoring (STM, previously presented), mediated by Complement Mediated Signal Amplification (CMSA) and the Membrane Assisted Complement Mediated Signal Amplification (MACMSA), also achieve such by the use of a signal generation process initiated by an immunogenic protein or other immunogenic target and permits no signal to be generated by a non-specific target analyte. All Haystack Processing strategies result in direct analysis of previously non-analyzable large samples of analyte with the ability to detect the presence of very low copy number DNA, RNA, cell subsets, and protein and other immunogenic targets of interest.

The MACMSA process provides a complement fixation assay for immunogenic targets. The MACMSA assay has been presented earlier and is based upon C3a generation as a result of complement fixation and activation, wherein the increasing number of targets produces increasing levels of C3a.

### The TSE META SCREEN ASSAY: TARGETS FOR PrP^{SC} DETECTION IN BLOOD AND OTHER HOST FLUIDS EARLY IN The INFECTIOUS TIME-COURSE

As previously presented, the targets for PrP^{SC} presence, are indirect in origin, due to the lack of an antibody specific to the aberrant prion. Meta Screen Assay functions by detection of plasminogen, host primary antibody (IgM) and a complement fixation product C3b, C3d, C4b, or other complement cascade product or fragment on a PrP^{SC} molecule, not found on the PrP^{C} or PrP prion molecule.

This is achieved by use of an antibody-sensitized red blood cell (Rh Pos/R2R2), stromal cocktail, represented as follows:

| **TARGET** | **Molecule Pair to Sensitize Rh Pos Stroma for MACMSA** |
|---|---|
| Plasminogen (PGₙ) | IgG anti PGₙ - IgG anti-D |
| Host Primary Antibody to PrP^{SC} (low levels) *ex. bovine assay* | Heterologous IgG anti bovine IgM (or other Ab)-IgG anti-D |
| Complement fragment on PrP^{SC} | IgG anti C3b - IgG anti-D |
| | IgG anti C3d - IgG anti-D |
| | IgG anti C4b - IgG anti-D |
| | IgG anti other complement component - IgG anti-D |

### SENSITIZATION OF RBCs BY THE MOLECULE PAIR METHOD

This novel method for sensitization of RBCs uses the D antigen expressed homogeneously on the entire RBC surface to attach the molecule pair. The D antigen was selected due to its inability to fix complement upon complexation with the anti-D antibody of the molecule pair. In related applications, other sites on the RBC may also be used, however, since complement fixations must not occur, an Fc deficient Fab or (Fab)₂ fragment to another homogeneously surface expressed RBC protein or other similarly functioning moiety may be used in place of IgG anti-D.

The method for sensitization of the intact RBCs by the molecule pair has been presented in related applications. Sensitized RBC stromal may be produced from sensitized RBCs by any method known to those skilled in the art. One preferred example may involve the use of digitonin to produce high quality stroma.

### STRATEGIC APPROACHES TO THE SENSITIVE EARLY INFECTION DETECTION OF PrP^{SC} IN BLOOD

STRATEGY I: This process utilizes the existence of a monoclonal antibody specific for the PrP^{SC} that exhibits no reactivity to normal prion (PrP). To date, no monoclonal antibody has been found to selectively discriminate the aberrant (PrP^{SC}) from normal prion (PrP^{SC} or PrP). The steps of the process are:
   Step I: Isolation of all the prion in a sample (PrP and PrP^{SC}) by attachment to a magnetic bead coated with a charged moiety known to those skilled in the art, that will specifically bind this prion protein subset.
   Step II: Addition of the monoclonal antibody specific to PrP^{SC} in the form of sensitized RBC stroma (IgG anti PrP^{SC}-IgG anti-D is the sensitization molecule pair).
   Step III: Addition of the immune complement reagent and incubation under conditions to generate the amplified signal, namely, the C3a peptide.
   Step IV: Assay for the C3a peptide by any method known to those skilled in the art.
STRATEGY II: This process eliminates the need for a discriminatory monoclonal antibody for PrP and PrP^{SC}. It has been reported that the PrP^{SC}molecule binds plasminogen, the inactive zymogen proteolytic enzyme, while the PrP does not bind the zymogen. Furthermore, documented observations, previously discussed herein, also support the attachment of an IgM antibody to the aberrant prion. This IgM antibody would be produced as a low titer (possibly currently undetectable by current ELISA assay) primary, antibody response to a protein antigen, similar to a protein vaccine. Also, the model presented herein calls for complement fixation or C3b or other complement fragments to be attached to the prion/3 target complex presented. The steps of this process are:
   Step I: Isolation of all the prion in a sample (PrP and PrP^{SC}) by attachment to a magnetic bead coated with a charged moiety known to those skilled in the art, that will specifically bind this prion protein subset.
   Step II: Addition of the monoclonal antibody specific for any non-Kringle region site on the PGₙ molecule.
      It should be noted that plasminogen is known by those skilled in the art to participate in fibrinolysis, metastasis of cancer cells into tissue, and release of normal prion proteins from blood platelets, thereby insuring that the PrP^{SC} molecule possesses invasive potential as well as securing the flow of blood platelets and providing the continuous supply of PrP substrate for conversion to PrP^{SC} and the resultant production of the amyloid fibrils and plaques in brain tissue continuous in the disease time-course.
      One must also consider that plasminogen is prevalent in blood at high concentration and PrP^{SC} upon introduction to the body, due to reaction kinetics, based on concentrations of the reactants, should be spontaneously bound to the PrP^{SC} molecules present.
      Furthermore, antibody (monoclonal) specific to the host IgM and another antibody (monoclonal) specific to C3b may also be added. All three antibodies should indirectly detect the early time-course PrP^{sc}/PGₙ/host IgM/C3b complex.
   Step III: Addition of the immune complement reagent with cofactors and incubation under conditions to generate the amplified signal, namely, C3a peptide production.
   Step IV: Assay for the C3a peptide by any method known to those skilled in the art.
STRATEGY III: This process eliminates the need for use of the immune complement reagent, while still focusing on detection of the plasminogen moiety attached to the PrP^{sc} molecule, absent from the PrP molecule.
   Note: The activation peptide of the plasminogen post PrP^{SC} binding must be sterically available for removal by any activation molecule known to those skilled in the art.
   Step I: Isolation of all the prion in a sample (PrP and PrP^{sc}) by attachment to a magnetic bead coated with a charged moiety known to those skilled in the art, that will specifically bind this prion protein subset.
   Step II: Addition of an activation molecule under conditions that will convert the plasminogen zymogen proenzyme to the active enzyme plasmin, known by those skilled in the art.
   Step III: Addition of another zymogen or proenzyme that the plasmin will activate, or addition of a substrate that the plasmin will catalytically modify, both steps initiate generation of a signal. Any zymogen, cascade, or any enzymatic reactionknown to those skilled in the art may be used. The preceding is referred to as Zymogen Mediated Signal Amplification or ZMSA and was presented previously (provisional patent number 60/226,823).
   Step IV: Addition of a substrate to the second zymogen (proenzyme) to catalytically generate a detectable signal.
   Step V: Detection of the amplified signals, generated in Steps III and IV, by any method known to those skilled in the art.
STRATEGY IV: This process comprises use of steps involving serine proteases activating immune complement. The PrP^{sc} plasminogen complex is treated by any method known to those skilled in the art resulting in the formation of the active enzyme, plasmin. The resulting plasmin should activate the complement pathway by cleavage of C3 into C3 a pathway resulting in the increasing generation of the C3a signal peptide.
   Step I: Isolation of all the prion in a sample (PrP and PrP^{SC}) by attachment to a magnetic bead coated with a charged moiety known to those skilled in the art, that will specifically bind this plasma protein subset.
   Step II: Addition of any activation molecule, known by those skilled in the art, under conditions that will convert the plasminogen zymogen proenzyme to the active enzyme plasmin.
   Step III: Addition of immune complement reagent and cofactors under conditions that will allow the activation of complement, resulting in the generation of the amplified signal, the C3a peptide.
   Step IV: Assay for the C3a peptide by any method known to those skilled in the art.
STRATEGY V: Monoclonal antibodies currently available that bind, PrP^{SC}, PrP^{c} and PrP can be attached to magnetic bead and used to remove all prion proteins from the sample. This will support large sample testing and will allow thorough removal by washing of non-specific moieties associated with the prion fraction, such as plasma plasminogen, plasma IgM, and plasma C3b.
   This strategy will be fully presented as the Meta Screen Assay and will be automated on robotics platform.

### ADDITIONAL STRATEGIES FOR PrP^{SC} ASSAY DEVELOPMENT

The embodiments for the sensitive detection of PrP^{SC}presented, herein, are only meant to indicate a direction in assay development or treatments for humans or animals. Any similar strategy is herein included, and any other moiety, such as a different enzyme or another type of molecule that has specificity for PrP^{sc} and not PrP is also included herein. The present invention is illustrated by the examples included herein which are not to be construed in any way as imposing limitation. The present invention is further illustrated by the following examples, which are not to be construed in any way as imposing limitations upon the scope thereof. It will be clear to one of skill in the art that various other modifications, embodiments, and equivalents thereof exist which do not depart from the spirit of the present invention and/or the scope of the appended claims.

### THE MULTIPLEX BOVINE METASCREEN ASSAY

The sensitive C3a complement fixation MACMSA assay can not only detect low copy number aberrant prions, but may also include detection of other diseases of the host. One embodiment in cattle may provide early and sensitive multiplex detection for the following:

| **MULTIPLEX DISEASES** | **DISEASE AGENT** |
|---|---|
| PrP^{sc} (BSE) | Prion |
| Vesicular Stomatitis Virus (VSV) | Virus |
| Foot and Mouth Disease (FMD) | Virus |
| Mycobacteria Species | Bacterium |
| Brucellosis Species | Bacterium |

The main requirement for these and additional disease screening in this sensitive C3a complement fixation system is the production of the molecule pair sensitized RBC stroma. The following correlates the disease with the specific molecule pair sensitizing antibody:

| **DISEASE** | **TARGET** | **SENSITIZING MOLECULE PAIR** |
|---|---|---|
| BSE | PrP^{SC} | IgG anti PGₙ-IgG anti-D |
| BSE | PrP^{SC} | Mouse IgG anti bovine IgG - IgG anti-D |
| BSE | PrP^{SC} | IgG anti C3b - IgG anti-D |
| BSE | PrP^{SC} | IgG anti C3d-IgG anti-D |
| BSE | PrP^{SC} | IgG anti C4b - IgG anti-D |
| VSV | Protein Coat | IgG anti VSV coat protein-IgG anti-D |
| FMD | Protein Coat | IgG anti FMD coat protein-IgG anti-D |
| Mycobacteria | Cell Surface Protein | IgG anti mycobacterium surface protein-IgG anti-D |
| Brucellosis, etc. | Cell Surface Protein | IgG anti Brucella surface protein - IgG anti-D |

It need be understood that these diseases include any number of and all known, and the targets selected may be any known, as long as one possesses an accompanying antibody, antibody fragment, or other molecule with similar function.

This antibody specific for the target becomes one part of an antibody pair, a molecule pair; the other antibody of the pair is any antibody and related moieties previously discussed that functions to attach the molecular pair to any surface on the red blood cell or stroma to be sensitized. In a preferred embodiment this attachment site is the Rh POS (R2R2) surface protein on the RBC.

### THE BOVINE METASCREEN ASSAY (see Table V)

The targets of interest must interact with their respective sensitized RBC stroma to assure complement fixation and activation, monitored by C3a production.

### Step I

### A: Isolation Of All Prions

The first step involves isolation of all prion in the plasma sample by attachment of both PrP^{SC} and PrP^{c} (PrP) to antibody coated magnetic beads. The antibody removes all prion from the plasma and allows washing of the prion material (the Mab is specific to the C terminal end of all prion). Washing removes non-specifically bound plasminogen bovine IgM, and exogenous C3a, C3b, C3d and C4b from the plasma attached to the prion coated magnetic beads.

It must be remembered that plasminogen, endogenous host antibody (IgM) and C3a, C3b, C3d and C4b is found in normal plasma, necessitating washing all bound prions to remove these non-specific moieties.

### B: Plasma Aliquot Secured For Analysis

The remaining plasma contains bovine pathogenic viruses and bacteria, which is analyzed separately from the prions, but in parallel. This will be later described in detail.

The prions, after thorough washings, bound to the magnetic beads, are treated with the three RBC sensitized stromas previously mentioned. An aliquot of the remaining plasma is directly added to the four RBC sensitized stromas that relate to the specific virus and bacteria presence that were previously mentioned. These microorganisms may fix complement by either the classical pathway or the alternate pathway. Plasma containing intact microorganisms, upon introduction to the corresponding sensitized stroma (anti-microbe antibody is present), will result in complement fixation by the classical pathway; however, carbohydrate present on the microorganism may similarly fix complement via activation of the alternate pathway (note: activation of the alternate pathway also leads to C3 a production).

### Step II

### A: Release of Bound Prion Into A Suspension Of Sensitized RBC Stroma

The total prion content on the magnetic bead is released by any method known to those skilled in the art (ex. Chemical, acidity, alkalinity, treatment followed by bead removal and solution neutralization, etc.) into a solution containing the three sensitized stroma (specific to plasminogen, host IgM, C3b, C3d, and C3b to screen selectively for PrP^{SC} in a complement fixation MACMSA assay where PrP^{C} and PrP are transparent to the assay.

### B: Addition of Plasma Aliquot To A Suspension Of The Corresponding Sensitized RBC Stroma

This direct addition of a large plasma aliquot will allow the presence of the microorganism at low numbers to directly interact with the sensitized stroma and to directly interact with the sensitized stroma and to fix complement via the classical pathway, produce C3a, and be detected.

### Step III: Incubate And Add Human Complement

Initial formation of the antigen/antibody-stromal complex upon addition of complement and cofactors results in C3a peptide formation. This includes use of washed prion with its respective three molecule pair sensitized stromas as a separate sample. Also included is the use of plasma aliquot and complexation with the appropriate anti-microbial sensitized RBC stromas as another separately analyzed sample.

### THE META SCREEN BOVINE DIAGNOSTIC ASSAY

The Meta Screen Bovine diagnostic assay is presented in Table V and Table VI. The diagnostic process is a multiphase approach in a multiplex diagnostic assay. Herein, the Meta Screen Assay presented will involve bovine diagnostic multiplex assay to early detect a number of targets responsible for a number of disease states early in the bovine infection time-course. Similar Meta Screen Assays may be configured for a human multiplex diagnostic (including a target responsible for Creutzfeld Jacob Disease (TSE in humans), such as that responsible for the anti-prion antibody to ₙᵥCJD, as well as other targets such as pre-cancerous (dysplasia), cancer, metastatic cancer, residual disease and any other applicable and similar target

The Meta Screen Assay may also be converted into an environmental panel wherein, immunogenic chemical pollutants may be multiplex assayed along with other targets such as microbial contaminants, carcinogenic molecules, and any other conceivable and applicable target that meets the immunogenic criterion of the process, the immunogenic property of the target.

Meta Screen diagnostic panels may be configured for all biological organisms and their diseases that can be determined to have an immunogenic target and a monoclonal antibody specific for that target.

### THE MACMSA DIAGNOSTIC PROCESS: A COMPLEMENT FIXATON ASSAY (Table V and Table VI)

As a complement fixation assay based on a number of targets, MACMSA would reflect the extent of complement fixation known to those skilled in the art and as such allow quantification of the extent of signal amplification. These amplified products of complement fixation and activation could include all peptides produced by fixation and activation of the cascade as well as the formation of any of the cascade structures, or any other moiety produced by this fixation and activation.

It was recognized that an effective method to perform a complement fixation reaction, namely MACMSA would be to use a molecule pair comprised of two attached antibodies to sensitize RBC stroma as a mechanism to assure maximal complement fixation and activation of a single immunogenic target to be detected. It is known by those skilled in the art that a lipid matrix is required to assure maximal complement fixation and activation of a single immunogenic molecule.

The molecule pair, in one embodiment is a monoclonal IgG antibody specific to the target, coupled by known chemistries to another IgG antibody specific to the Rh D protein on the RBC, the site for attachment of the molecule pair and other possible attachment sites. The attachment moiety may also be an antibody fragment, or other attachment molecule specific for any homogeneously expressed protein on the surface of the RBC.

The D site of molecule pair attachment was selected due to the known inability of the D/IgG anti-D to fix complement. Thus molecule pair attachment will sensitize the RBC without chemical modification and without complement fixation or activation, along with the antibody fragments Fab or (Fab)₂, and similar functioning molecules can be used, providing complement is not fixed or activated.

The theoretical extent of amplification or complement fixation employing this system has been calculated to be 40,000 C3a peptides produced per single immunogenic molecule target in MACMSA, described earlier herein. Theoretically delivering a BSE diagnostic assay sensitive to 100 aberrant prion molecules (4 million C3a molecules are readily detectable by any method known to those skilled in the art).

### DESCRIPTION OF THE BOVINE META SCREEN ASSAY: PHASE I (Table V)

The goal of the assay is to determine the presence of the predicted prion/protein complex that would be found early in the infection. This complex as stated is PrP^{SC}/plasminogen/host IgM antibody/C3b, C3d, and/or C4b.

Confirmation of any component of this complex will provide a sensitive diagnostic result

### Step I: The Sample (Aliquot I)

Plasma from a single animal or pools of plasma from several animals may be used as a sample. An aliquot of 100 µl can be assayed on a robotic analyzer device The ability to analyze a large volume sample is known by those skilled in diagnostics to assure the detection of the pathologic target in the earliest stages of the host infection time-course.

If necessary for early BSE detection, a 50cc or larger plasma sample can be manually prepared and the concentrated prions introduced into the robotic platform for automated PrP^{SC} complex target detection. To achieve this, magnetic beads would be added to the large volume plasma sample.

### Step II: Magnetic Bead Addition

Magnetic beads are added that are coated with an IgG monoclonal antibody that or any other molecule known to bind PrP^{sc}, PrP^{c}, and PrP, namely all prion forms, known by those skilled in the art.

### Step III: Wash

Wash the magnetic beads to remove non-specific proteins including plasminogen, human antibody (or any type, specifically IgM in the circulation or other intestinal antibody.

### Step IV: Dissociate the Prions

Any method known by those skilled in the art may be used to dissociate the prions from the magnetic beads, place them in a separate tube and remove the stripped magnetic beads.

### Step V: Sensitized Stroma Addition

Add molecule pair sensitized Rh Pos (R2R2) sensitized stroma. The R2R2 Rh Pos type was selected for its greatest RBC surface expression and strongest interaction with the anti-D moiety of the molecule pair. Any other Rh Pos type may also be used. The molecule pairs are:
IgG anti PGₙ - IgG anti-D
IgG anti IgM (or other Ab) - IgG anti-D
IgG anti C3b - IgG anti-D
IgG anti C3d - IgG anti-D
IgG anti C4b - IgG anti-D

The attachment site of the molecule pair should be homogeneously expressed on the RBC surface. The above are the preferred embodiment but all other similarly functioning attachment molecules have been discussed.

The molecule pairs presented will detect the aberrant prion protein complex (PrP^{SC}/plasminogen/bovine IgM/any complement fragment attached) found early in the BSE time-course.

### Step VI: Incubate To Allow The Antigen/Antibody Reaction To Occur

Incubate 37°C for 30 to 60 minutes at pH 7.2.

### Step VII: Complement and Cofactor Addition

Complement, calcium and magnesium are added to the prion-stroma mixture. One embodiment calls for use of human complement due to the fact that the C3a peptide ELISA used to quantify C3a is composed of monoclonal antibodies (capture and reporter) specific to the human C3a peptide. Calcium and magnesium are each added at a concentration of 20 mM.

### Step VIII: Incubate at Room Temperature

Incubate at 24°C for 30 to 60 minutes to allow complement fixation by the target/stroma complex and activation resulting in increased or amplified levels of C3a production.

### Step IX: Assay for C3a Peptide

The prion/stroma/complement mix is mixed by periodic aspiration up and down in the pipette tip and, upon completion of the incubation, the stroma is allowed to settle to the well bottom; an aliquot of supernate is removed of 50 to 100 µl to quantify C3a peptides present.

Quidel Corporation possesses a C3a (human) sandwich ELISA using horseradish peroxidase to generate a color substrate signal.

Another C3a quantification assay embodiment currently under development is a C3a sandwich ELISA using magnetic beads coated with the capture antibody IgG anti C3a and the reporter antibody another IgG anti C3a labeled with biotin (for example), and subsequent use of a streptavidin alkaline phosphatase conjugate, which, coupled with a sensitive chemiluminescent substrate (1,2 Dioxetane) known to those skilled in the art, provides a more sensitive assay for C3a quantification.

### DESCRIPTION OF THE BOVINE META SCREEN ASSAY: PHASE II (Table V)

The goal, herein, is to detect the presence of microbes, bacteria, fungi, virus, etc. in the sample. The complement fixation reaction involves the presence of immunogenic proteins found on the microbe surface and requires the possession of the IgG anti-microbe surface protein molecule attached to the IgG anti-D (the molecule pair) used to sensitize the stroma.

### Step I. The Sample (Aliquot II)

Plasma from a single animal or pools of plasma from several animals may be used as a sample. Currently, an aliquot of 100 µl can be assayed on a robotic analyzer devise. The ability to analyze a large volume sample is known by those skilled in diagnostics to assure the detection of the pathologic target in the earliest stages of the infection time-course.

If necessary for early BSE detection, a 50cc or larger plasma sample can be manually prepared and the microbes present can be concentrated by any method known to those skilled in the art. The concentrated microbes are nextprocessed by direct addition to the various sensitized stromas.

### Step II: Sensitized Stroma Panel Addition

The sensitized stroma panel, in this embodiment, will detect the presence of Vesicular Stomatitis Virus (VSV), Foot and Mouth disease virus (FMD), Mycobacterial species, Brucella species and even the Nemavirus that some believe to be the etiologic agent for BSE (Narang, 1998). The molecule pairs used to sensitize the Rh Pos (R2R2) RBC stroma are:
IgG anti VSV coat protein - IgG anti-D
IgG anti FMD coat protein - IgG anti-D
IgG anti any Mycobacteria surface protein - IgG anti-D
IgG anti any Brucella surface protein - IgG anti-D
IgG anti any Nemavirus surface protein - IgG anti-D

The sensitized stromal panel will support the complement fixation reactions to detect the presence of the intact microbe.

### Step III: Incubate To Allow The Microbe/Antibody Reaction To Occur

Incubate 37°C for 30 to 60 minutes at pH 7.2.

### Step IV: Complement and Cofactor Addition

Complement, calcium, and magnesium are added to the microbe-stroma mixture. One embodiment calls for use of human complement due to the fact that the C3a peptide ELISA used to quantify C3a is composed of monoclonal antibodies (capture and reporter) specific to the human C3a peptide. Calcium and magnesium are each added at a concentration of 20 mM.

Heterologous complement from other species may be used, only if the two anti-C3a monoclonals for the species specific C3a peptide are in hand.

### Step V: Incubate at Room Temperature

Incubate the mixture at 24°C for 30 to 60 minutes to allow complement fixation by the target/stroma complex and activation resulting in increased or amplified levels of C3a production.

### Step VI: Assay for C3a Peptide

The microbe/stroma mix is mixed by periodic aspiration and, upon completion of the incubation, the stroma is allowed to settle to the well bottom; an aliquot of supernate is removed of 50 to 100 µl to quantify C3a peptides present.

Quidel Corporation possesses a C3a peptide sandwich ELISA using horseradish peroxidase to generate a color substrate signal.

Another C3a quantification assay embodiment currently under development is a C3a sandwich ELISA using magnetic beads coated with the capture antibody IgG anti C3a and the reporter antibody another IgG anti C3a labeled with biotin (for example), and subsequent use of a streptavidin alkaline phosphatase conjugate, which, coupled with a sensitive chemiluminescent substrate (1,2 Dioxetane) known to those skilled in the art, provides a more sensitive assay for C3a quantification.

### DESCRIPTION OF THE BOVINE META SCREEN ASSAY: PHASE III (Table V)

The goal, herein, is to confirm detection of the presence of a microbe in the sterile plasma of the cow. This approach is based on the complement activation produced by the Alternate. Complement Pathway in the absence of the antibody on the sensitized stroma. It is known by those skilled in the art that intact microbes possess carbohydrate surface moieties, which when exposed to complement, with its cofactors activates complement and produces C3a peptides proportionate to the number of microbes present in the sample.

This internal confirmation can detect low copy microbial targets in any fluid, such as plasma, urine, cerebrospinal, and amniotic.

### Step I: The Sample (Aliquot III)

Plasma from a single animal or pools of plasma from several animals may be used as a sample. An aliquot of 100 µl can be assayed on a robotic analyzer devise. The ability to analyze a large volume sample is known by those skilled in diagnostics to assure the detection of the pathologic target in the earliest stages of the infection time-course.

If necessary for early BSE detection, a 50cc or larger plasma sample can be manually prepared and the microbes present can be concentrated by centrifugation, dialysis, or any other method known to those skilled in the art. The concentrated microbes are next processed by direct addition of complement.

The goal of this phase of the assay is to confirm the presence of the target microbe by another signal amplification method, comprising direct activation of the Alternate Complement Pathway by the direct presence of the microbe, in the sterile sample, resulting in increased C3a peptide production

### Step II: Complement and Cofactor Addition

Complement, containing properdin and Factor B as cofactors, is directly added to the plasma sample for analysis. Interaction of the microbe and complement will activate complement and result in C3a peptide production. One embodiment of the assay calls for the use of human complement, due to the fact that the C3a peptide ELISA, used to quantify C3a is composed of monoclonal antibodies (capture and reporter) specific to the human C3a peptide.

### Step III: Incubate at Room Temperature

Incubate the mixture at 24°C for 30 to 60 minutes to allow complement activation (no fixation due to lack of Ag/Ab complex formation) resulting in increased or amplified levels of C3a production.

### Step IV: Assay for C3a Peptide

The microbe/stroma composite is mixed by periodic aspiration up and down in the pipette tip and, upon completion of the incubation, the stroma is allowed to settle to the well bottom; an aliquot of supernate is removed of 50 to 100 µl to quantify C3a peptides present

Quidel Corporation possesses a C3a peptide sandwich ELISA using horseradish peroxidase to generate a color substrate signal.

Another C3a quantification assay embodiment currently under development is a C3a sandwich ELISA using magnetic beads coated with the capture antibody IgG anti C3a and the reporter antibody another IgG anti C3a labeled with biotin (for example), and subsequent use of a streptavidin alkaline phosphatase conjugate, which, coupled with a sensitive chemiluminescent substrate (1,2 Dioxetane) known to those skilled in the art, provides a more sensitive assay for C3a quantification.

### DESCRIPTION OF THE BOVINE META SCREEN ASSAY: PHASE IV

In related applications, an automated analysis technique has bee presented for automated RNA isolation and direct RNA analysis using RP-TFO RNA TPA diagnostic analysis process.

It is the goal of this phase of the assay to confirm on a molecular level (mRNA) the presence of the organism and even determine the serotype, and determine the antibiotic resistance of the microbe, to initiate a successful treatment course.

This RP-TFO diagnostic process works well with single stranded mRNA or other single stranded RNA targets, such as found in VSV and FMD infections. Furthermore, mRNA can be detected for any derepressed gene in the DNA of the microbe, for example, the mRNA, which would establish antibiotic resistance to the microbe.

### THE META SCREEN BOVINE MULTIPLEX DIAGNOSTIC ASSAY

**TABLE VI**

| **DIRECT RNA ANALYSIS** |
|---|
| **PHASE IV** |
| Plasma (Aliquot 4) |
| Automated Magnetic Bead RNA Extraction |
| |
| Perform RP-TFO RNA TPA) |
| Add |
| RP-TFO Specific for RNA of: VSV FMD |
| |
| *Perform* signal amplification *MTRF:* |
| *Sensitive signal* |
| *Signal amplification process* |
| *Create a chemiluminescent signal* |
| |
| **Assay for chemiluminescence** |
| Disease Screen: |
| Vesicular Stomatitis Virus, |
| Foot and Mouth Disease |

### THERAPEUTIC STRATEGY IN LIMITING AND RESOLVING BSE INFECTION ROLE OF THE MOLECULE PAIR IN VIVO: DISEASE ERRADICATION

### HOST ANTIBODY RESPONSE TO PrP^{SC} AND ITS CONSEQUENCE

With the presented evidence that B cells and T cells participate in an early TSE (BSE) infection event (described and referenced herein), one scheme for BSE and PrP^{SC} infection would involve antigen (PrP^{SC}) DISPLAY by the appropriate T lymphocyte and subsequent presentation to a B cell subset that would produce an antibody specific to PrP^{SC}.

This antibody would be required to fix complement, a requirement also found in an early infection event in scrapies (described and referenced herein). It could be an IgM or other antibody that is characterized as a single molecule that alone can fix complement. Any other similar single antibody molecules fixing complement are included in embodiments of the invention.

The complement requirement would place complement fragments C3b, C3d, C4b and other molecules on the PrP^{SC}/plasminogen/host antibody complex.

It is known by those skilled in the art that all phagocytic cells possess one of a number of complement fragment receptors, primarily functioning in opsonized clearance of antigen/antibody complexes and microbial organisms, which are both opsonized in vivo. The following chart presents phagocytic cell types, all possessing the C3b receptor for internalization of the antigen, which in this discussion is the PrP^{SC}/plasminogen/host antibody/complement fragment complex:

| **Phagocytic Cell Type** (Different Phagocytic compartments) | **Type** | **Location Of Phagocytic Activity** | **Involvement Of Tissue In BSE Infection Time-Course** |
|---|---|---|---|
| Polymorphonuclear Granulocyte | Polys or PMNs | Peripheral blood | Not postulated |
| MATURE Monocyte (fixed monocytes) | Spleen | Spleen | B-cells in the spleen |
| | Liver | Liver | Kupfer cells, fixed tissue monocyte expressing C3b |
| | Intestine | Intestine | M-Cell , a fixed tissue monocyte expressing C3b receptor, M cells/ vaccine effector site |
| | Brain | Brain | Glial Cells or fixed tissue monocyte expressing C3b |
| IMMATURE Monocyte | Peripheral blood | Peripheral blood | May occur to a minor extent |

Ingestion of the PrP^{SC} complex would carry the target complex throughout the lymphatic system, the tonsils, and the other lymphoid organs to the brain.

Now that the PrP^{SC} complex has left the intestine and traveled to the brain via the lymphatic system, amyloid fibril formation begins to occur and plaques begin to form characteristic of the disease.

The singular point of disruption of the proposed time-course is the phagocytic event itself, no matter where it occurs. It is known that some viruses, such as HIV, form low grade infections in monocytes (fixed tissue and immature circulating types), survive and advance the disease state. It is also understood that a similar virus phagocytized and internalized by a different phagocytic cell subset namely the circulating polymorphonuclear granulocytes would be destroyed. This is a function of different digestive vesicles and enzymes being present in different phagocytic compartments.

In this BSE infection scenario and this invension, it is stated that the C3b mediated PrP^{sc} complex internalization is a critical point to disrupt the infection and limit and resolve it.

It is proposed that a shift in the phagocytic cell compartment for the PrP^{sc} complex may result in its internalization and destruction. To achieve this, a method is needed to direct the PrP^{sc} complex away from the fixed monocytes in tissues to the circulating polymorphonuclear granulocytes in the peripheral blood.

### USE OF A MOLECULE PAIR SENSITIZED BOVINE RED BLOOD CELL TO CHANGE THE PHAGOCYTIC COMPARTMENT WHICH INTERNALYZES THE PrP^{sc} COMPLEX

A therapeutic embodiment of the molecule pair has been presented in related applications where it has been proposed that immunogens in the circulation can be removed from the circulation mediated by a method for attachment of the immunogen in vivo to the molecule pair sensitized RBCs and that the nature of this attachmentexclusively predisposes the phagocytosis by the polymorphonuclear granulocytes in circulation as opposed to the fixed tissue monocytes regulated primarily by the dispersal of the sensitizing antibody on the RBC surface, represented by the following chart:

| **Molecule Pair Attachment Sites on Sensitized RBC Surface** | **Mechanism** | **Site of Ingestion** |
|---|---|---|
| Discrete CRI Patches around surface of RBC | CR1 exchange reaction (Taylor, 1998) | Fixed monocytes spleen/liver |
| Homogeneous over entire surface of RBC | PMN phagocytosis | Circulating PMNs |

### DESIGN OF THE MOLECULE PAIR FOR A STRATEGY TO DIRECT THE PrP^{SC} COMPLEX TO A DIFFERENT PHAGOCYTIC COMPARTMENT TO RESULT IN ITS DESTRUCTION

The molecule pair is characterized as being a pair of monoclonal antibody molecules covalently attached, one being specific for the target, namely the PrP^{SC} complex and the other specific for a site that is homogeneously expressed on the bovine RBC surface.

It is known that the life expectancy of a bovine RBC is approximately four months and a number of proteins are homogeneously expressed on the surface. The molecule pair attachment site would be any one that is homogeneously expressed over the entire surface.

The attachment antibody cannot fix complement, and as such, an antibody fragment (Fab) or (Fab)₂ devoid of the Fc region would be used to avoid complement fixation and sensitized RBC clearance by the circulatory system. The antibody recognizing the PrP^{sc} complex would be the IgG anti complement fragment antibody, identical in function to the complement receptors on the phagocytic cell surface. The molecule pairs of the preferred embodiment of the invention would be: IgG anti C3b - (Fab)₂ anti bovine RBC antigen, anti C3d - (Fab)₂ anti bovine RBC antigen, anti C4b - (Fab)₂ anti bovine RBC antigen, and similar for any other complement fragment attached to the PrP^{sc} complex.

### PrP^{sc} CLEARANCE FROM THE BLOODSTREAM

The stated molecule pair is used to in vivo sensitize the bovine RBCs (4 month life expectancy). The IgG anti complement fragment antibody attached to the RBC surface will compete with the monocytic phagocytic cells for the PrP^{SC}/plasminogen/host antibody/complement fragment complex and understanding that the sensitized RBCs would greatly outnumber the fixed tissue monocytes, one can prevent the PrP^{SC} complex internalization by the "normal" route and attach the complex to sensitized RBCs via the molecule pair. This will interrupt the normal BSE infection course.

It is also known to those skilled in the art that polymorphonuclear granulocytic phagocytes also express Fc receptors on their surface, which aid in intrnalization of any complex containing the Fc region. This should further assist in interruption of the normal infection course of the PrP^{SC} complex. In reality, this may not play an important role in phagocytic compartment redirection probably do to the presence of Fc receptors in the fixed monocytes of the spleen and liver, which are responsible for destruction of antibody coated RBCs (Fc regions present).

Next, the RBCs with attached PrP^{SC} complex are phagocytized as the complexes bind to the molecule pair sensitized RBCs. The zipper mechanism known for PMN phagocytosis attaches the PMN to the first PrP^{SC} complex which remains intact in the circulation to mop up or attaché additional PrP^{SC} complexes, causing more complete phagocytosis until a certain threshod of PrP^{SC} complexes is met, which supports the total engulfment and destruction of the sensitized RBC and all the PrP^{SC} complexes that are attached.

It is important to note that, one, the PrP^{SC} complex is effectively neutralized upon binding to the sensitized RBC and no downside exists for its delayed destruction, and two, binding of the PrP^{SC} complex to the molecule pair sensitized RBC will not fix additional complement and release toxic hemoglobin into the circulatory system, due to the presence of decoy acceleration factors (DAF) found on blood elements and body tissues that prevent formation of the membrane attack complex (MAC), a group of late complement cascade proteins that form a hole in the cell membrane. DAF on the RBC membrane inhibits MAC formation.

The mechanism of action of DAF involves the acceleration of destruction of classical and alternate pathway C3 and C5 convertases (Nicholson-Weller, 1982; Medof, 1984; Pangburn, 1986; Fujita, 1987).

This new phagocytic compartment would prevent the PrP^{SC} complex from pursuing its "normal" course and the PrP^{SC} complex internalization in the granulocytic, phagocytic compartment would destroy the complex and limit resolve the infection, as well as any other different compartment involved in internalization and digestion of the PrP^{SC} complex.

### References:

1. Agostoni, A., et al. Activation of complement and kinin systems after thrombolytic therapy in patients with acute myocardial infarction: a comparison between streptokinase and recombinant tissue-type plasminogen activator. Circulation 1994 Vol. 90:2666-2670
2. Aguzzi, et al. Diagnostics and therapeutics for transmissible spongiform encephalopathy and methods of manufacture of non-infective blood products and tissue derived products. 1998 PCT Patent No.WO 99/30738
3. Brade, V., et al. Formation of the C-3 cleaving properdin enzyme on zymosen. J. Immunology 1974 Vol. 113:1735-1743
4. Bronstein, I, Brooks, E, Voyta JC. 1,2-Dioxetanes: Novel Chemiluminescent Enzyme Substrates. Application to Immunoassays. J Bioluminescence & Chemiluminescence 1989 Vol. 4: 99-111
5. Bruce, ME, Will RG, Ironside JW, et al. Transmissions to mice indicate that "new variant" CJD is caused by the BSE agent. Nature 1997 Oct. Vol. 389:498
6. Christensen, U., C-terminal lysine residues of fibrinogen fragments essential for binding to plasminogen. FEBS Lett. 1985 Vol. 182:43-36
7. Cochrane, C.G., et al. Activation of Hageman factor in solid and fluid phases. J. Exp. Med. 1974 Vol. 138:1564-1583
8. Denson, K.W. & Redman, C.W. The destruction of factor VIII clotting activity by thrombin and plasmin. Thromb. Res. 1980 Vol. 18:547-549
9. Edmonds-Alt, X., et al. Proteoglycan and fibrin-degrading neutral proteinase activities of Lewis lung carcinoma cells. Eur. J. Cancer 1980 Vol. 16:1257-1261
10. Eigen, M. Prionics or the kinetic basis of prion diseases. Biophys. Chem. 1996 Vol. 63:A1-A18
11. Eklund, et. al. Pathogenesis of scrapie virus infection in the mouse. J. Infect. Dis. 1967 Vol. 117:15-22
12. Fischer, M.B., et al. Binding of disease-associated prionprotein to plasminogen. Nature 2000 Vol. 408:479-483
13. Fujita, T. et al. The mechanism of action of decay-accelerating factor (DAF). DAF inhibits the assembly of C3 convertases by dissociating C2a and Bb. J. Exp. Med. 1987 Vol. 166:1221-1228
14. Gross, J.L., et al. Increased capillary endothelial cell protease activity in response to angiogenic stimuli in vitro. Proc. Natl Acad. Sci. USA 1983 Vol. 80:2623-2627
15. Habal, F.M., et al. Generation of kinin by plasma kallikrein and plasmin and the effect of α1-antitrypsin and antithrombin III on the kininogenases. Adv. Exp. Med. Biol. 1976 Vol. 70:23-36
16. Hamanoue, M., et al. Plasminogen binds specifically to α-enolase on rat neuronal plasma membrane. J. Neurochem. 1994 Vol. 63:2048-2057
17. Hortin, G.L., et al. Alpha 2-antiplasmin's carboxy-terminal lysine residue is a major site of interaction with plasmin. Biochem. Biophys. Res. Commun. 1988 Vol.155:591-596
18. Janeway, C.A., et al. Intravenous γ-globulin: Mechanism of gamma-globulin fragments in normal and agamma-globulinemic persons. N. Engl. J. Med. 1968 Vol. 278:919-923
19. Jilek, F. Cold-insoluble globulin III. Cyanogens bromide and plasminolysis fragments containing a label introduced by transamidation. Z. Physio. Chem. 1977 Vol. 358:1165-1168
20. Khalil, N., et al. Plasmin regulates the activation of cell-associated latent TGF-β1 secreted by rat alveolar macrophages after in vivo bleomycin injury. Am. J. Respir. Cell Mol. Biol. 1996 Vol. 15:252-259
21. Kuhn, S.E., Taylor, R.P., et al. Escherichia coli Bound to the primate erythrocyte complement receptor via bispecific monoclonal antibodies are transferred to and phagocytosed to by human monocytes in an in vitro model. The J. of Immunology 1998 Vol. 160:5088-5097
22. Lee, C.D. & Mann, K.G. Activation/inactivation of human factor V by plasmin. Blood 1989 Vol. 73:185-190
23. MacKay, A.R. et al. Basement membrane type IV collagen degradation: evidence for the involvement of a proteolytic cascade independent of metalloproteinases. Cancer Res. 1990 Vol. 50:5997-6001
24. Medof, M.E., et al. Inhibition of compliment activation on the surface of cells after incorporation of decay-accelerating factor (DAF) into their membranes. J. Exp. Med.1984 Vol. 160:1558-1578
25. Miyato, T., et al. Plasminogen Tochigi: Inagctive plasmin resulting from replacement of Ala600 by Thr at the active site. Proc. Natl Acad. Sci USA 1982 Vol. 79:6132-6136
26. Morioka, S., et al. Migrating keratinocytes express urokinase-type plasminogen activator. J. Invest. Dermatol. 1991: Vol. 88:418-423
27. Mucosal Immunology-2nd ed., Pearay L. Ogra (Editor), Jerry R> McGhee (Editor) Warren Strober (Editor), John BIenenstock (Editor), Michael Lamm (Editor), Jiri Mestecky (Editor), Publish Date September 1998
28. Mullin, D.E. & Rohrlich, S.T. The role of proteinases in cellular invasiveness. Biochim. Biophys. Acta 1983 Vol. 695:177-214
29. Narang, H.K. Evidence that single-stranded DNA wrapped around the tubulofilamentus particles termed "nemaviruses" is the genome of the scrapie virus. Res. Virology 1998 Vol. 149:375-382
30. Nicholson-Weller, A. et al. Isolation of the human erythrocyte membrane glycoprotein with decay-accelerating activity for C3 convertases of the complement system. J. Immunology 1982 Vol. 129:184-189
31. Ossowski, L. & Reich, E., Antibodies to plasminogen activator inhibit human tumor metastasis. Cell 1983 Vol. 35:611-619
32. Ossowski, L., et al. Mammary plasminogen activator: Correlation with involution hormonal modulation and comparison between normal and neoplastic tissue. Cell 1979 Vol. 16:929-940
33. Pangbum M.K. Differences between the binding sites of the complement regulatory proteins DAF, CR1 and factor H on C3 convertases. J. Immunol. 1986 Vol. 136:2216-21
34. Reich, R., et al. Follicular plasminogen activator: involvement in ovulation. Endocrinology 1985 Vol. 116:516-521
35. Safar, J, Wille, H, Itri, V, et al. Eight prion strains have PrPsc molecules with different conformations. Nature Medicine 1998 Oct. Vol.4 (10):1157-1165
36. Schlechte, W. et al. Examination of the role of the urokinase receptor in human colon cancer mediated laminin degradation. Cancer Res. 1989 Vol. 49:6064-6069
37. Strickland, S., et al. Plasminogen activator in early embryogenesis: Enzyme production by trophoblast and parietal endotherm. Cell 1976 Vol. 9:231-240
38. Stricklin, G.P., et al. Human skin collagenase: Isolation of precursor and active forms from both fibroblast and organ cultures. Biochemistry 1977 Vol. 16:1607-1615
39. Sugiyama, N., et al. Binding site of α1-plasmin inhibitor to plasminogen. Iochim. Biophys. Acta. 1988 Vol.952:1-7
40. Unkeless, J.C., et al. An enzymatic function associated with transformations of fibroblasts by oncogenic viruses. J. Exp. Med. 1973 Vol. 137:85-111
41. Varadi, K., et al. Activation and inactivation of human protein C by plasmin. Thromb. Haemost. 1994 Vol. 71:615-621
42. Virgi, M.A.G., et al. Plasminogen activator of islets of Langerhans: modulation by glucose and correlation with insulin production. 1980 Proc. Natl Acad. Sci. USA 1980 Vol. 77:875-879

## Claims

1. Use of RBCs capable of binding pathogenic organisms for the preparation of a medicament, means or remedy for clearing a human or animal of pathogenic organisms.

2. Use according to claim 1, wherein the RBCs capable of binding pathogenic organisms are antibody sensitized RBCs.
